Europäisches Patentamt

European Patent Office

Office européen des brevets

(11) Publication number: **0 369 316**
**A2**

# EUROPEAN PATENT APPLICATION

(21) Application number: 89120754.0

(22) Date of filing: 09.11.89

(51) Int. Cl.5: **C12N 15/62, C12N 15/26, C12N 15/31, A61K 37/02**

(30) Priority: 17.11.88 US 272356

(43) Date of publication of application:
23.05.90 Bulletin 90/21

(84) Designated Contracting States:
AT BE CH DE ES FR GB GR IT LI LU NL SE

(71) Applicant: F. HOFFMANN-LA ROCHE AG
Postfach 3255
CH-4002 Basel(CH)

(72) Inventor: Ju, Grace W.
2 Ann Street, Unit S218
Clifton, N.J. 07013(US)

(74) Representative: Mezger, Wolfgang, Dr. et al
Grenzacherstrasse 124 Postfach 3255
CH-4002 Basel(CH)

(54) Recombinant interleukin-2 hybrid proteins.

(57) Production of recombinant human interleukin-2 (IL-2) fusion proteins can be substantially enhanced by utilizing a vector construction capable of expressing the hybrid protein at high levels. Additionally, the resulting protein can be purified with high yield employing a receptor-affinity column. A further advantage of the present vector construction is that the IL-2 expressed has a more natural sequence than the product provided by prior known expression systems for producing IL-2 hybrid molecules. In a specific embodiment of the invention, a fusion protein of IL-2 and Pseudomonas exotoxin is expressed at high levels in E. coli and purified to homogeneity with excellent recoveries and the desired high biological activity.

EP 0 369 316 A2

## Recombinant Interleukin-2 Hybrid Proteins

The present invention relates to hybrid proteins composed of a human interleukin-2 (IL-2) polypeptide and a polypeptide acting as an immunotoxin. Such fusion proteins are useful as therapeutic agents for the treatment of diseases such as autoimmune thyroiditis, type 1 diabetes, rheumatoid arthritis and Crohn's disease, where cells bearing IL-2 receptors (IL-2R positive cells) are thought to be involved in the aberrant regulation of the immune response. These immunotoxic fusion proteins are also useful for suppressing the immune response in cases of rejection of allografts and organ transplants.

Lorberboum-Galski et al., Proc. Natl. Acad. Sci. USA, 85, 1922-1926 (1988) have disclosed the cloning and expression of the chimeric protein $Asp^2IL2$-Pseudomonas exotoxin ($Asp^2IL2$-PE40) in E. coli. In this fusion protein, IL-2 replaces the cell recognition domain at Pseudomonas exotoxin (PE), or in other words the fusion protein has a recombinant human interleukin-2 sequence at the amino terminus and the carboxyl terminus of said fusion protein comprises the amino acid sequence of a PE molecule lacking domain I. A PE molecule from which domain I has been deleted (= PE 40) has full ADP-ribosylating activity but extremely low cell killing activity because of loss of the cell recognition domain. The IL-2 molecule at the amino terminus of the fusion protein is fully exposed and retains its biological activity and provides a different cell recognition domain than the one present in PE. As a result of the procedure for preparing the chimeric protein, an aspartic acid residue is inserted between the alanine and proline at positions 1 and 2 of the IL-2 molecule thus rendering the IL-2 part of the molecule "un-natural" at the amino terminus. A synthetically encoded isoleucine is used to link the threonine at position 131 of IL-2 to the amino terminus of the toxin molecule, i.e. PE40 (proline at position 251 of PE). A further description of the production of the $Asp^2IL2$-PE fusion protein is set forth in European Patent Application, Publication No. 261 671, published March 30, 1988. The $Asp^2IL2$-PE40 fusion protein displays cytotoxic activity for cells bearing IL-2 receptors.

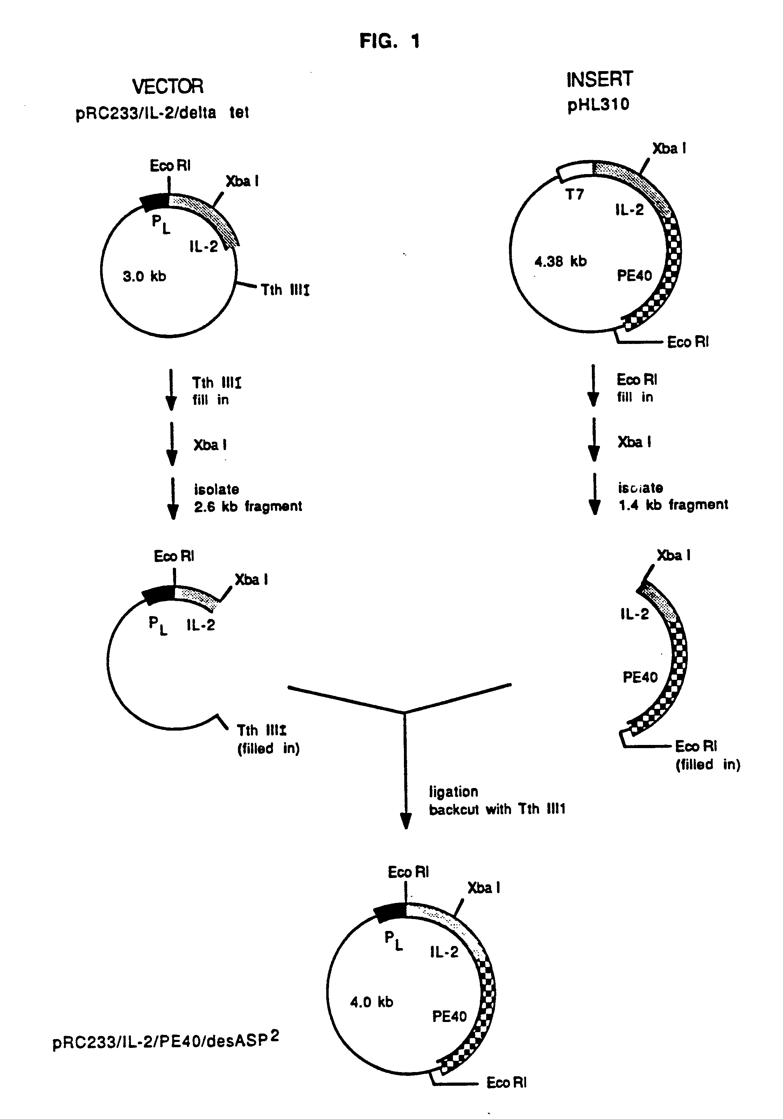

The vector pHL310 used by Lorberboum-Galski et al., (supra) for the expression of the $Asp^2IL2$-PE fusion protein lead to a peptide with the amino terminal sequence Met-Ala$^1$Asp$^2$Pro$^3$Thr$^4$... which differs from the sequence found in natural human IL-2 (Robb et al., Proc. Natl. Acad. Sci. USA 81, 6486-6490, [1984]). Moreover the vector pHL310 did not allow the expression of high levels of protein. To correct these two problems, the strategy set forth in Figure 1 was employed to create an expression vector that would program the synthesis of very high levels of the fusion protein, and would code for a fusion protein with an amino terminus that was more similar to that of human IL-2, i.e. Met-Ala$^1$Pro$^2$Thr$^3$....

Thus, the present invention provides a recombinant human IL2-PE immunotoxin hybrid protein distinguished in having a natural IL-2 sequence at its amino terminus as compared to the $Asp^2IL2$-PE40 fusion protein described in the art. The immunotoxin hybrid protein has a recombinant human interleukin-2 sequence at the amino terminus and the carboxyl terminus of said hybrid protein comprises the sequence of a domain of Pseudomonas exotoxin, preferably Pseudomonas exotoxin 40, characterized in that the amino terminal sequence of said interleukin-2 is identical with the amino terminal sequence of natural human interleukin-2. The immunotoxin hybrid protein may optionally comprise an additional methionine residue at the N-terminus. Most preferably the immunotoxin hybrid protein is in essentially homogeneous form. Moreover the present invention provides a DNA coding for such an immunotoxin hybrid protein and a recombinant vector comprising such a DNA. Most preferably the recombinant vector is a plasmid. A unicellular host organism transformed with such a recombinant vector or plasmid is capable of expressing a normalized human IL2-PE hybrid protein. Preferably the unicellular host organism is a conventional bacterial host, such as, most preferably, E. coli.

The parental vector chosen for constructing the plasmid described in the Example is derived from the known plasmid pRC233/IL-2/delta tet, which is a plasmid of about 3 kilobasepairs (kb) that contains the complete cDNA coding sequence of human IL-2 downstream (3') of the regulatory sequences of the bacteriophage lambda $P_L$ promoter. The plasmid pRC233/IL-2/delta tet is capable of expressing recombinant human IL-2 in an E. coli host at high levels. The plasmid is described in European Patent Application, Publication No. 147 819, published July 10, 1985. A further disclosure of the preparation and use of this plasmid to produce recombinant human IL-2 is provided by Ju et al. in J. Biol. Chem. 262, 5723-5731 (1987).

Thus, the hybrid proteins of the present invention are produced using methods of the recombinant DNA technology. Thereby a unicellular host organism containing a recombinant vector comprising a DNA sequence coding for the said hybrid protein is fermented i.e. cultured under conditions suitable for the expression of the said DNA sequence and the hybrid protein produced by the unicellular organism is isolated from the culture.

Such unicellular host organism may be a prokaryotic or a eukaryotic cell. A large number of such unicellular host organisms are commercially available or freely available from depositories such as the American Type Culture Collection (ATCC) located at 12301 Parklawn Drive, Rockville, Maryland, USA. Examples of prokaryotic cells are bacteria such as E.coli [e.g. E.coli M15 described as DZ291 by Villarejo et al. in J. Bacteriol. 120, 466-474 [1974], E.coli 294 (ATCC No. 31446), E.coli RR1 (ATCC No. 31343) or E.coli W3110 (ATCC No. 27325)], bacilli such as B.subtilis and enterobactericeae among which can be mentioned as examples Salmonella typhimurium and Serratia marcescens. Especially preferred is E.coli strain MC1061 containing the plasmid pRK248clts (see Example). Alternatively yeast cells such as Saccharomyces cerevisiae may be used as host organisms. A large number of eukaryotic cells suitable as host organisms for recombinant vectors are known to the man skilled in the art. Mammalian cells such as CV-1 (ATCC No. CCL 70) and derivatives thereof such as COS-1 (ATCC No. CRL 1650) or COS-7 (ATCC No. CRL 1651) are preferably used. In addition, it is possible to use insect cells such as those described by Smith et al. (Mol. Cell. Biol. 2, 2156-2165 [1983]).

Various methods for introducing recombinant vectors into unicellular host organisms are known. Examples for such methods are microinjection, transfection or transduction. The man skilled in the art has no difficulty to select the most suitable method for the specific host organism used.

The recombinant vectors used in the present invention are vectors containing a DNA coding for an immunotoxin hybrid protein of the present invention. Such a DNA may be synthesized by conventional chemical methods, e.g. by the phosphotriester method which is described by Narang et al. in Meth. Enzymol. 68, 90-108 [1979], or by the phosphodiester method (Brown et al., Meth. Enzymol. 68, 109-151 [1979]). In both methods long oligonucleotides are first synthesized and then joined together in a predetermined way. The nucleotide sequence of the DNA may be identical to the natural nucleotide sequences or may be partially or completely different. This is due to the fact that the genetic code is degenerate, that means that one amino acid may be coded by several codons. The codons selected may be adapted to the preferred codon usage of the host organism used to express the immunotoxin hybrid protein (Grosjean et al., Gene 18, 199-209 [1982]). Care must be taken that the DNA obtained in this way does not contain partial sequences which make the construction of the recombinant vector difficult, e.g. by introducing an undesired restriction enzyme cleavage site, or which prevents the expression of the polypeptide.

A large number of vectors such as those mentioned in European Patent Application, Publication No. 200 986 may be used for constructing the recombinant vectors mentioned above. These vectors contain elements necessary for transcription and translation of the DNA coding for the immunotoxin hybrid protein as well as elements needed for the maintenance and replication of the vector in the host. The selection of a suitable vector for constructing the recombinant vectors of the present invention and the selection of a unicellular host organism compatible with said recombinant vectors is within the skills of an artisan in the field.

Preferably the recombinant vectors of the present invention are prepared by modifying recombinant vectors containing a DNA coding for an immunotoxin hybrid protein using the oligonucleotide directed site specific mutagenesis method described by Morinaga et al., Bio/Technology 2, 636-639 (1984). An example for such a recombinant vector comprising a DNA coding for an immunotoxin hybrid protein is the plasmid pHL310 mentioned above.

The manner in which the expression of the immunotoxin hybrid protein in accordance with the invention is effected depends on the expression vector and on the host organism used. Usually, the host organisms which contain the expression vector are grown up under conditions which are optimal for the growth of the host organism. Towards the end of the exponential growth, when the increase in the number of cells per unit time decreases, the expression of the immunotoxin hybrid protein is induced, i.e. the DNA coding for the protein is transcribed and the transcribed mRNA is translated. The induction can be effected by adding an inducer or a derepressor to the growth medium or by altering a physical parameter, e.g. by a temperature change.

The immunotoxin hybrid protein produced in the host organism can be secreted by the cell by special transport mechanisms or can be isolated by breaking open the cell. The cell can be broken open by mechanical means (Charm et al., Meth. Enzmol. 22, 476-556 [1971]), by enzymatic treatment (e.g. lysozyme treatment) or by chemical means (e.g. detergent treatment, urea or guanidine•HCl treatment, etc.) or by a combination thereof.

In eukaryotes, polypeptides which are secreted from the cell are synthesized in the form of a precursor molecule. The mature polypeptide results by cleaving off the so-called signal peptide. As prokaryotic host organisms are not capable of cleaving eukaryotic signal peptides from precursor molecules, eukaryotic polypeptides must be expressed directly in their mature form in prokaryotic host organisms. The translation start signal AUG, which corresponds to the codon ATG on the level of the DNA, causes that all polypeptides are synthesized in a prokaryotic host organism with a methionine residue at the N-terminus. In certain cases, depending on the expression system used and possibly depending on the polypeptide to be expressed this N-terminal methionine residue is cleaved off.

The immunotoxin hybrid protein of the present invention can be purified to essential homogeneity by known methods such as, for example, by centrifugation at different velocities, by precipitation with ammonium sulphate, by dialysis (at normal pressure or at reduced pressure), by preparative isoelectric focusing, by preparative gel electrophoresis or by various chromatographic methods such as gel filtration, high performance liquid chromatography (HPLC), ion exchange chromatography, reverse phase chromatography and affinity chromatography (e.g. on Sepharose™ Blue CL-6B or on carrier-bound IL-2R or monoclonal antibodies directed against IL-2 or Pseudomonas exotoxin).

The purified immunotoxin hybrid protein of the present invention can be employed in a manner known per se for the treatment of diseases such as autoimmune thyroiditis, type 1 diabetes, rheumatoid arthritis, Crohn's disease and other diseases where an aberrant regulation of the immune response is thought to be involved. The immunotoxin hybrid protein is also useful for suppressing the immun response in cases of rejection of allografts and organ transplants.

The immunotoxin hybrid proteins prepared in accordance with this invention may be administered to warm blooded mammals for the clinical uses indicated above. The administration may be by any conventional method auch as by parenteral application either intravenously, subcutaneously or intramuscularly. Obviously, the required dosage will vary with the particular condition being treated, the severity of the condition, the duration of the treatment and the method for administration. A suitable dosage form for pharmaceutical use may be obtained from sterile filtered, lyophilized protein reconstituted prior to use in a conventional manner. It is also within the skill of the artisan in the field to prepare pharmaceutical compositions containing an immunotoxin hybrid protein of the present invention by mixing the said immunotoxin hybrid protein with compatible pharmaceuti cally acceptable carrier materials such as buffers, stabilizers, bacteriostats and other excipients and additives conventionally employed in pharmaceutical parenteral dosage forms. The present invention also relates to such pharmaceutical compositions.

Having now generally described this invention, the same may be more readily understood by reference to the following example when considered in connection with the following drawings, wherein

Figure 1 shows schematically (not drawn to scale) the construction of the plasmid of the present invention used for the expression of a fusion protein designated IL2-PE40, comprising a human IL-2 having an amino terminus corresponding to the sequence of authentic human IL-2 and a desired toxin domain of PE, i.e., PE40.

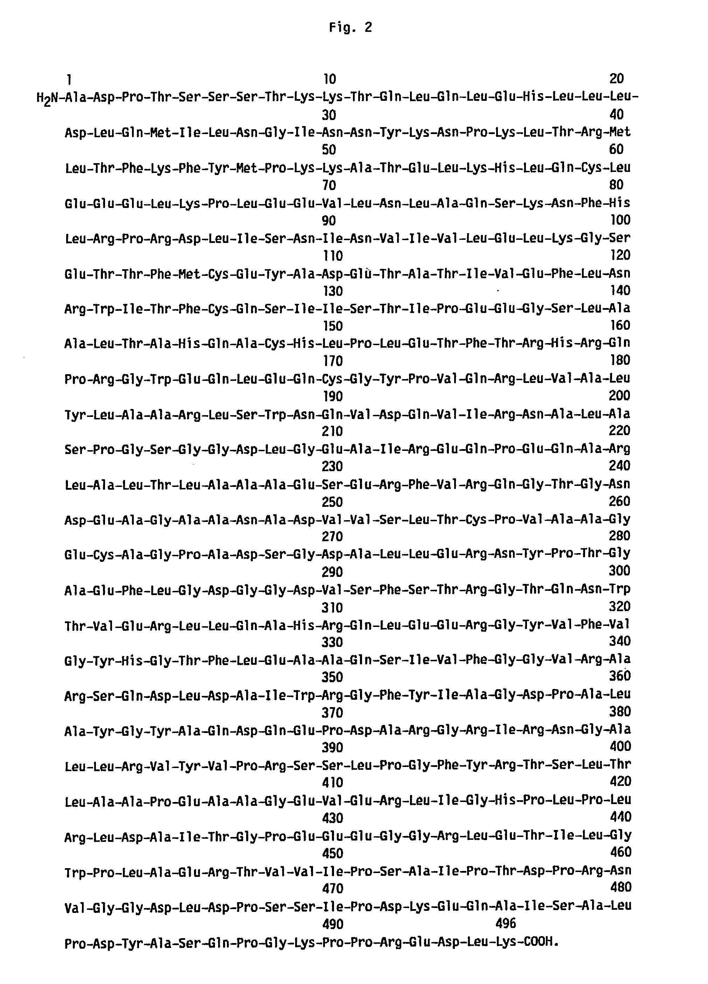

Figure 2 depicts the amino acid sequence of $Asp^2IL2$-PE hybrid protein as predicted from the DNA sequence of the cDNA clone utilized in the preparation of the plasmid pHL310 of the prior art.

Figure 3 shows the first 15 amino acid residues obtained upon sequencing of the IL2-PE40 hybrid protein produced by expression of the $pRC233/IL-2/PE40/desASP^2$ plasmid of the present invention.

Figure 4 depicts a scheme for the purification of IL2-PE40.

It should be understood that the following example is for illustrative purposes only and should no be construed as limiting this invention in any way to the specific embodiment recited therein.

## Example

20 micrograms of pRC233/IL-2/delta tet DNA were digested with Tth 111 I (New England Biolabs, Beverly, Mass., U.S.A.), then filled in with 4 deoxyribonucleotide triphosphates (dNTPs) using Klenow DNA polymerase (Bethesda Research Laboratories, Bethesda, Maryland, U.S.A.). After phenol extraotion, the DNA was digested to completion with Xba I (Boehringer Mannheim, Mannheim, BRD). The filled in 2.6 kb Xba I/Tth 111 I fragment was isolated by elution after electrophoresis through low-melting agarose purified using an Elutip column (Schleicher and Schuell, Keene, New Hamspshire, U.S.A.). About 2 micrograms of vector DNA was recovered.

The insert fragment was derived from the plasmid pHL310 (European Patent Application, Publication No. 261 671). This plasmid contains the $Asp^2IL2$-PE40 sequences located downstream of the bacteriophage T7 promoter. 20 micrograms of pHL310 DNA were digested to completion with Eco RI (New England Biolabs), then filled in with 4 dNTPs and Klenow DNA polymerase. After phenol extraction, the DNA was digested to completion with Xba I. The 1.4 kb fragment was purified as described above for the vector fragment. About 1.2 micrograms of insert DNA were recovered.

To construct the recombinant expression vector, 320 nanograms of the 1.4 kb fragment (pHL310, Xba

l/Eco RI filled in) were mixed with 50 nanograms of the vector DNA (pRC233/IL-2/delta tet, Tth 111 I filled in). These DNAs were ligated in PL buffer (6.5 mM Tris-HCL, pH 7.4, 0.1 M NaCl, 4.5 mM MgCl$_2$, 100 mM 2-mercaptoethanol) in the presence of 1.0 mM ATP and T$_4$ DNA ligase (New England Biolabs). The ligation reaction was incubated at 15°C for 2 hours. Additional T$_4$ DNA ligase was included in the reaction and the mixture was then incubated at 4°C for 18 hours. The ligation mixture was heated at 65°C for 5 minutes to inactivate the ligase, then digested with Tht 111 I to linearize any remaining parental vector DNA. A portion of the ligation mixture was then transformed into competent E. coli MC1061 cells containing the plasmid pRK248clts. The E.coli strain MC1061 and the plasmid pRK248clts are available from the American Type Culture Collection under the accession numbers ATCC No. 53338 and ATCC No. 33766, respectively. The host strain E. coli MC1061 and the compatible plasmid pRK248clts, which codes for the temperature-sensitive repressor of the P$_L$ promoter, are known to the man skilled in the art and have been described by Casadaban et al., J. Mol. Biol. 138, 179-207 [1980] and by Bernard et al., Methods Enzymol. 68, 482-492 [1979], respectively. The E.coli cells were made competent according to the procedure described by Maniatis et al., in "Molecular Cloning; A Laboratory Manual", Cold Spring Harbor Laboratory U.S.A., pp. 249-255 [1982].

The resultant colonies were screened for the correct recombinant vector by minilysate preparation of plasmid DNA (Maniatis et al., supra, pp. 366-369) and restriction digestion with Eco RI, Xba I, and Bgl II. Two colonies containing the appropriate plasmid (designated pRC233/IL-2/PE40/desASP[2]) were further analyzed for protein production. Cultures from the two colonies (designated #4 and #5) were grown at 30°C until OD$_{600}$ = 0.5, and then shifted to 42°C until OD$_{600}$ = 1.0 to induce protein synthesis. Pellets of the induced cells were extracted by lysis in Laemmli sample buffer (Laemmli, Nature 227, 680-685 [1970]) as described by Ju et al., supra. Analysis of the pellets by polyacrylamide gel electrophoresis indicated that both cultures produced the expected protein of 54 kilodaltons, which was not present in uninduced cultures. The extract from clone #4 was also found to contain substantial bioactivity as assayed with murine CTLL and other cells. For a detailed description of the assay used and the results obtained can be found below.

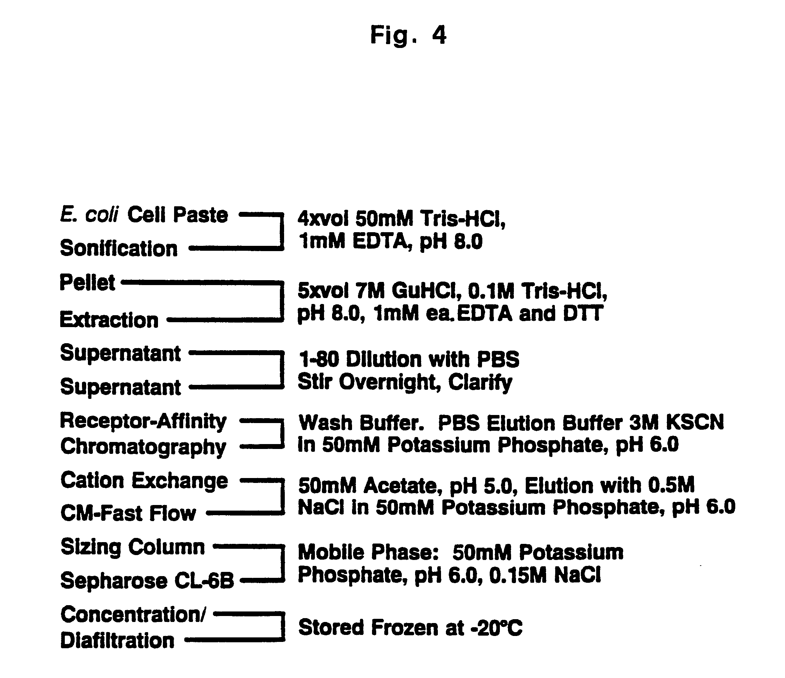

The culture of clone #4 containing the plasmid pRC233/IL-2/PE40/desASP[2] was used to seed a large 20 liter fermentation batch. Cells from this fermentation were treated as described to extract and purify the IL2-PE40 fusion protein. The overall purification scheme for IL2-PE40 is shown in Figure 4.

For solubilization and renaturation steps all operations were carried out at 2-8°C, unless indicated otherwise. The frozen E. coli cells were thawed and suspended in 50 mM Tris-HCl, pH 8.0 containing 1 mM EDTA (4 ml/g cells). The cell suspension was pulse-sonicated 6 x 30 seconds with a Sonifier Cell Disruptor 350 (Branson Sonic Power Co., Farmingdale, New York, U.S.A.). The disrupted cells were centrifuged at 10'000 x g for 20 minutes in a Sorvall RC-5 centrifuge (Dupont, Wilmington, Delaware, U.S.A.) with a SS-34 rotor and the pellet was collected. The pellet was suspended in 5 ml/g cells of a solution of 7 M guanidine HCl (GuHCl) in 0.1 M Tris-HCl, pH 8.0 containing 1 mM each of EDTA and dithiothreitol (DTT) and sonicated as before. The mixture was stirred for 60 minutes and the supernatant was collected by centrifugation. It was diluted 80-fold with PBS (phosphate buffered saline, pH 7.4, Whittaker M.A. Bioproducts, Walkersville, Maryland, U.S.A.) and stirred overnight. The diluted extract was clarified by centrifugation at 24'000 x g for 20 minutes with GSA rotor or filtered through a 0.8/0.2 μ filter.

The clarified extract was used as the starting material for the receptor-affinity purification of IL2-PE40, followed by cation exchange and size-exclusive liquid chromatography. The IL2-PE40 activity in E. coli extract quantitatively binds to the receptor column as indicated by the absence of activity in the flow through materials. Approximately 98% of the bound IL2-PE40 activity is recovered from the receptor column. Gel permeation data, SDS-PAGE analysis under reducing and non-reducing conditions as well as bioassay results have indicated the presence of reducible and biologically inactive IL2-PE40 "aggregates" in the receptor-affinity purified material along with the major biologically active soluble monomeric form of IL2-PE40. When the receptor-affinity purified product is dialyzed against pH 5.0 buffer, most of the inactive materials precipitated. Cation exchange chromatography removes a strongly UV absorbing ($\lambda_{max}$ = 260 nm) non-proteinacious contaminant, which fails to bind to the cation exchange column. This step results in a specific activity increase. The IL2-PE40 preparation is free of high molecular weight contaminants as judged by SDS-PAGE under non-reducing conditions. Although no significant increase in purify was observed, gel permeation is useful in removing low molecular weight contaminants and endotoxin. The final preparation contains no detectible level of endotoxin, when the gel filtration step is carried out under aseptic conditions. This step is also a convenient way of exchanging the IL2-PE40 into the final storage buffer.

The procedures for the production, and purification of a soluble form of IL-2R(p55), denoted IL-2R-Δ-Nae have been described by Hakimi et al., J. Biol. Chem. 262, 17336-17341 [1987]. The purified IL-2R was immobilized to NuGel P-AF Poly-N-hydroxy-succinimide (500 Å, 50 μm, Separation Industries, Metuchen, New Jersey, U.S.A.) at a coupling density of 1.05 mg/ml gel and used as the affinity adsorbent for IL-2PE40 purification as described by Bailon et al., Bio/Technology 5, 1195-1198 (1987).

Forty milliliters of immobilized IL-2R gel were packed into an Amicon G-22 x 250 column fitted with two adapters (Amicon, Div. W.R. Grace & Co., Danvers, Mass., U.S.A.). The column was equilibrated with phosphate buffered saline (PBS). 7 liters of extract (1/2 of the total extract derived from 35 g cells) was applied to the column at a flow rate of 7 ml/minutes. The column effluents were monitored by a Gilson 111B UV detector and recorded by a Kippen Zonen recorder (Gilson Medical Electronics, Inc., Middletown, Wisconsin, U.S.A.). The column was washed with PBS until the absorbance at 280 nm returned to the baseline. The IL2-PE40 activity was eluted from the column with 3 M KSCN in 50 mM potassium phosphate, pH 6.0. The stock KSCN solution was decolorized by filtering through an activated charcoal filter, prior to use. The eluted IL2-PE40 was dialyzed against 2 liters of 50 mM sodium acetate, pH 5.0 in 12-14,000 M.W. cut-off dialysis tubings. The dialysate was centrifugated to remove any precipitated materials. The clear dialysate was now ready for cation exchange chromatography.

The dialyzed IL2-PE40 from the previous step was applied to a 1.6 x 15 cm CM-Fast Flow column (Pharmacia LKB Biotechnology Inc., Piscataway, New Jersey, U.S.A.), equilibrated with 50 mM sodium acetate, pH 5.0 at a flow-rate of 3.3 ml/minutes. The column was washed with the equilibration buffer until all the unadsorbed materials were removed. The adsorbed IL2-PE40 was eluted from the column with 0.5 M NaCl in 50 mM potassium phosphate, pH 6.0. It was then concentrated in a thin channel stirred cell Amicon concentrator fitted with a YM 10 membrane.

A Pharmacia K25/100 column was packed with Sepharose™ CL-6B (Pharmacia LKB Biotechnology Inc.) to a height of 90 cm. The column was equilibrated with 50 mM potassium phosphate, pH 6.0 containing 0.15 M NaCl, at a flow rate of 0.7 ml/minutes. The concentrated IL2-PE40 (20 ml) was applied to the column and developed with the equilibration buffer. The column operations were monitored as described in the affinity step . Six-minute fractions were collected in an LKB Ultra Rac-7000 fraction collector (LKB-Produkter, Brömmer, Sweden). The peak corresponding to the IL2-PE40 activity (~54 kDa) was collected and concentrated to 0.5-1.0 mg protein per ml, filtered through a 0.2 $\mu$ filter and stored frozen at -20° C. The gel permeation step was carried out under aseptic conditions.

The bioactivity of IL2-PE40 of the present invention may be examined in vitro by studying its ability to inhibit protein synthesis in lymphoid cells bearing high affinity receptors for IL-2. The cells which may be used in these studies include cells of the murine CTLL line (CTLL is a long-term, IL-2-dependent murine T cell line available from ATCC under accession number TIB 214), murine splenocytes which had been activated to express high affinity IL-2 receptors (IL-2R) by incubation with concanavalin A (Con A), and human peripheral blood lymphocytes which had been activated to express high affinity IL-2R by incubation in mixed leukocyte cultures (MLC blasts). As a negative control any cells which lack IL-2R can be used such as the murine P815 mastocytoma line (ATCC No. TIB 64).

Murine Con A-activated splenocytes can be prepared by incubating splenocytes from C57BL/6 mice at a density of $1 \times 10^6$ cells/ml in culture medium containing 5% heat inactivated fetal bovine serum and 2 $\mu$g/ml Con A. After incubation for 3 days at 37° C, the splenocytes were harvested from the cultures and washed 3 times in fresh medium without Con A prior to use in the assay. To prepare human MLC blasts, peripheral blood mononuclear cells from 2 normal volunteer donors are isolated as described by Gately et al., J. Natl. Can. Inst. 69, 1245-1254 (1982). The leukocytes from one donor ("stimulator cells") are treated with 1500 rads of X-irradiation prior to mixing with leukocytes from the second donor ("responder cells"). Stimulator cells ($1 \times 10^6$/ml) and responder cells ($1 \times 10^6$/ml) are cultured together for a total of 6 days at 37° C. On day 4 of culture, recombinant human IL-2 (rIL-2) is added to each culture at a final concentration of 50 units/ml. On day 6 the cells are harvested and washed 3 times in fresh medium prior to assay.

Assays were performed by mixing 50 $\mu$l aliquots of cells, rIL-2 and IL2-PE40 in the wells of flat-bottom microplates (e.g. Costar 3596). The medium used in the assays was leucine-free Eagle's minimal essential medium supplemented with 5% heat-inactivated fetal bovine serum, 0.1 mM nonessential amino acids, 1 mM sodium pyruvate, 2 mM L-glutamine, $5 \times 10^{-5}$ M 2-mercaptoethanol, 100 units/ml penicillin, 100 $\mu$g/ml streptomycin, and 50 $\mu$g/ml gentamicin. Human and murine lymphoblasts were added at a concentration of $2 \times 10^4$ cells/well, and murine cell lines were used at $5 \times 10^3$ cells/well. The cultures were incubated for 24 hours (CTLL and P815) or 48 hours (human and murine lymphoblasts) at 37° C. Twenty-five $\mu$l aliquots of $^3$H-lecuine ($^3$H-leu), 20 $\mu$Ci/ml, were then added to each well. The cultures were incubated for an additional 6 hours at 37° C and subsequently harvested onto glass fiber filters. $^3$H-lecuine incorporation into cellular protein was measured by liquid scintillation counting. All values reported are the means ± 1 SEM of triplicate samples.

The result of incubating IL2-PE40 with murine CTLL cells, which bear high affinity IL-2R (IL-2R[+]), and with murine P815 mastocytoma cells, which lack IL-2R (IL-2R[−]), are shown in Table 1. IL2-PE40 was a very potent inhibitor of protein synthesis by the IL-2R-bearing CTLL cells. Fifty percent inhibition of protein synthesis by CTLL cells was observed at concentrations of IL2-PE40 between 0.03 ng/ml and 0.16 ng/ml in these experiments. On the other hand, approximately 5 to 6 µg/ml of IL2-PE40 was required to cause 50% inhibition of protein synthesis by P815 cells, which lack IL-2R. Hence, there was a greater than 10'000-fold difference in the concentrations of IL2-PE40 which were toxic for an IL-2R-bearing murine cell line and an IL-2R-negative cell line in these experiments. This demonstrates that IL2-PE40 was selectively toxic for cells bearing high affinity IL-2R.

Table 1

| Abilities of IL2-PE40 and Asp$^2$IL2-PE40 to Inhibit Protein Synthesis by IL-2R$^+$ Murine CTLL and IL-2R$^-$ Murine P815 | | | | |
|---|---|---|---|---|
| rIL-2 (µg/ml) | IL2-PE40 (ng/ml) | Asp$^2$ IL2-PE40 (ng/ml) | $^3$H-Leu Incorporation into: | |
| | | | CTLL | P815 |
| Expt. 1 | | | | |
| 0 | 0 | 0 | 233 ± 58 | |
| 10 | 0 | 0 | 67,965 ± 2241 | |
| 10 | 4 | 0 | 197 ± 117 | |
| 10 | 0.80 | 0 | 2,103 ± 582 | |
| 10 | 0.16 | 0 | 11,841 ± 700 | |
| 10 | 0.03 | 0 | 23,712 ± 2829 | |
| 10 | 0 | 4 | 312 ± 337 | |
| 10 | 0 | 0.80 | 2,225 ± 544 | |
| 10 | 0 | 0.16 | 26,560 ± 1467 | |
| 10 | 0 | 0.03 | 60,218 ± 5415 | |
| 0 | 0 | 0 | | 52,338 ± 3890 |
| 0 | 50,000 | 0 | | 600 ± 183 |
| 0 | 10,000 | 0 | | 16,860 ± 1919 |
| 0 | 2,000 | 0 | | 36,849 ± 1862 |
| 0 | 400 | 0 | | 50,784 ± 4038 |
| 0 | 0 | 50,000 | | 990 ± 181 |
| 0 | 0 | 10,000 | | 17,314 ± 420 |
| 0 | 0 | 2,000 | | 40,219 ± 1264 |
| 0 | 0 | 400 | | 53,087 ± 1813 |
| Expt. 2 | | | | |
| 0 | 0 | 0 | 5 ± 51 | |
| 10 | 0 | 0 | 48,626 ± 3643 | |
| 10 | 4 | 0 | 419 ± 355 | |
| 10 | 0.80 | 0 | 3,666 ± 295 | |
| 10 | 0.16 | 0 | 23,775 ± 1312 | |
| Expt. 3 | | | | |
| 0 | 0 | 0 | 1,223 ± 219 | |
| 10 | 0 | 0 | 47,873 ± 2150 | |
| 10 | 4 | 0 | 94 ± 17 | |
| 10 | 0.80 | 0 | 2,962 ± 126 | |
| 10 | 0.16 | 0 | 21,903 ± 25 | |
| 10 | 0.03 | 0 | 32,825 ± 2307 | |
| 0 | 0 | 0 | | 31,987 ± 3009 |
| 0 | 10,000 | 0 | | 6,089 ± 409 |
| 0 | 2,000 | 0 | | 21,409 ± 844 |
| 0 | 400 | 0 | | 28,730 ± 1022 |

Table 2 shows the results of additional studies in which the effects of IL2-PE40 on primary murine or human lymphoblasts were examined. IL2-PE40 inhibited protein synthesis by Con A-activated murine splenocytes with 50% inhibition observed at approximately 4 ng/ml IL2-PE40. Similarly, IL2-PE40 inhibited protein synthesis by human MLC blasts with a 50% inhibitory concentration of approximately 11 ng/ml.

Table 2

| Abilities of IL2-PE40 to Inhibit Protein Synthesis by Con A-activated Murine Splenocytes and Human Lymphoblasts Activated in Mixed Leukocyte Cultures | | | | |
|---|---|---|---|---|
| rIL-2 ($\mu$g/ml) | IL2-PE40 (ng/ml) | Asp[2] IL2-PE40 (ng/ml) | $^3$H-Leu Incorporation into: | |
| | | | Murine Con A Blasts | Human MLC Blasts |
| Expt. 1[a] | | | | |
| 0 | 0 | 0 | 763 ± 273 | |
| 10 | 0 | 0 | 18,135 ± 936 | |
| 10 | 500 | 0 | 1,521 ± 96 | |
| 10 | 100 | 0 | 2,765 ± 50 | |
| 10 | 20 | 0 | 4,579 ± 154 | |
| 10 | 4 | 0 | 8,977 ± 352 | |
| 10 | 0.8 | 0 | 14,703 ± 892 | |
| 10 | 0 | 500 | 1,900 ± 191 | |
| 10 | 0 | 100 | 2,533 ± 84 | |
| 10 | 0 | 20 | 4,386 ± 70 | |
| 10 | 0 | 4 | 10,030 ± 570 | |
| 10 | 0 | 0.8 | 14,761 ± 1364 | |
| Expt. 2[a] | | | | |
| 0 | 0 | 0 | | 1,837 ± 198 |
| 10 | 0 | 0 | | 18,744 ± 511 |
| 10 | 500 | 0 | | 1,740 ± 199 |
| 10 | 100 | 0 | | 4,384 ± 180 |
| 10 | 20 | 0 | | 7,450 ± 624 |
| 10 | 4 | 0 | | 12,186 ± 822 |
| 10 | 0.8 | 0 | | 17,198 ± 859 |

[a] Data shown in this table under experiments 1 and 2 are from the same experiments as experiments 1 and 2, respectively, in Table 1.

In the experiments mentioned above the biological activity of IL2-PE40 was compared to that of the Asp[2]IL2-PE40 of the prior art, in assays on murine CTLL, P815, and Con A-activated splenocytes (Tables 1 and 2). The biological activities of IL2-PE40 and Asp[2]IL2-PE40 were very similar in these studies.

A sample of IL2-PE40 as prepared above was submitted to amino acid analysis using an instrument with postcolumn fluorescamine derivatization (see Pan and Stein, Amino acid analysis with postcolumn fluorescamine derivatization, in: "Methods of protein microcharacterization", Shively, J.E. (ed.), The Humana Press Inc., Clifton, New Jersey, U.S.A. [1986]). Prior to analysis, the protein was hydrolyzed in 6 N HCl containing 4% thioglycolic acid at 110° C for 20-24 hours in vacuo. The results are summarized in Table 3.

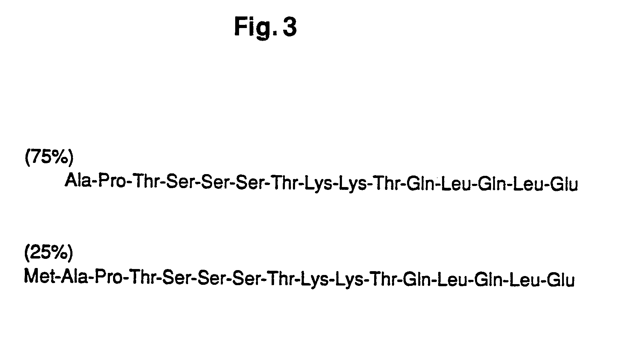

The sample was also submitted to sequence analysis. The two sequences shown in Figure 3 were obtained. 25% of the recombinant protein in the sample were found to contain an additional methionine residue at the N-terminus. It was found that the N-terminal amino acid sequence (the first 15 amino acids, disregarding the N-terminal methionine residue of one sequence) matches with the N-terminal amino acid sequence of natural human IL-2. It has to be noted that the results shown in Table 3 are based on a preliminary analysis of the sample. No buffer blank was analyzed as a control and PRO, CYS, and TRP were not determined (ND).

Table 3

| AMINO ACID ANALYSIS OF IL-2 PE40 | | |
|---|---|---|
| AMINO ACID | CALCULATED | DETERMINED |
| ASP (D) | 40 | 40.0 |
| THR (T) | 29 | 27.6 |
| SER (S) | 27 | 25.5 |
| GLU (E) | 65 | 64.2 |
| PRO (P) | 32 | ND |
| GLY (G) | 41 | 40.7 |
| ALA (A) | 51 | 51.6 |
| CYS (C) | 7 | ND |
| VAL (V) | 24 | 23.1 |
| MET (M) | 4 | 4.0 |
| ILE (I) | 23 | 21.1 |
| LEU (L) | 60 | 63.3 |
| TYR (Y) | 14 | 16.2 |
| PHE (F) | 15 | 16.0 |
| HIS (H) | 9 | 9.1 |
| LYS (K) | 14 | 15.5 |
| ARG (R) | 34 | 33.3 |
| TRP (W) | 6 | ND |

## Claims

1. An immunotoxin hybrid protein having a recombinant human interleukin-2 sequence at the amino terminus and the carboxyl terminus of said hybrid protein comprising the sequence of a domain of Pseudomonas exotoxin, characterized in that the amino terminal sequence of said interleukin-2 being identical with the amino terminal sequence of natural human interleukin-2 and optionally comprises an additional methionine residue at the N-terminus.

2. The immunotoxin of claim 1, wherein the said domain of Pseudomonas exotoxin at the carboxyl terminus is Pseudomonas exotoxin 40.

3. The immunotoxin of claim 1, wherein said immunotoxin is an essentially homogeneous protein.

4. A DNA coding for an immunotoxin hybrid protein as defined in claim 1 or 2.

5. A recombinant vector comprising a DNA coding for an immunotoxin hybrid protein as defined in claim 1 or 2.

6. A unicellular host organism containing a recombinant vector comprising a DNA coding for an immunotoxin hybrid protein as defined in claim 1 or 2.

7. An immunotoxin hybrid protein according to any one of claims 1 to 3 for the treatment of autoimmune diseases such as autoimmune thyroiditis, type 1 diabetes, rheumatoid arthritis or Crohn's disease and for suppressing the immune response in cases of rejection of allografts and organ transplants.

8. A process for the preparation of an immunotoxin hybrid protein according to claims 1 to 3 which process comprises:

(a) fermenting unicellular host organisms transformed with an expression plasmid capable of expressing the said immunotoxin hybrid protein at high levels; and

(b) purifying the said immunotoxin hybrid protein by procedures known in the art.

9. The process of claim 8, wherein the expressed hybrid immunotoxin protein is purified using an interleukin-2 receptor affinity chromatography step.

10. A pharmaceutical composition comprising a minor, effective amount of an immunotoxin hybrid protein as claimed in any one of claims 1 to 3 and a major amount of a pharmaceutical adjuvant suitable for parenteral administration.

11. Use of an immunotoxin hybrid protein according to any one of claims 1 to 3 for the treatment of autoimmune diseases such as autoimmune thyroiditis, type 1 diabetes, rheumatoid arthritis or Crohn's disease and for suppressing the immune response in cases of rejection of allografts and organ transplants.

12. Use of an immunotoxin hybrid protein according to any one of claims 1 to 3 for the preparation of a pharmaceutical composition according to claim 10.


Claims for the following Contracting States: GR, ES

1. A process for the preparation of a immunotoxin hybrid protein having a recombinant human interleukin-2 sequence at the amino terminus and the carboxyl terminus of said hybrid protein comprising the sequence of a domain of Pseudomonas exotoxin characterized in that the amino terminal sequence of said interleukin-2 is identical with the amino terminal sequence of natural human interleukin-2 and optionally comprises an additional methionine residue at the N-terminus which process comprises:

(a) fermenting unicellular host organisms transformed with an expression plasmid capable of expressing the said immunotoxin hybrid protein; and

(b) purifying the said immunotoxin hybrid protein by procedures known in the art.

2. A process for the preparation of an immunotoxin hybrid protein according to claim 1 wherein the said domain of Pseudomonas exotoxin at the carboxyl terminus is Pseudomonas exotoxin 40 which process comprises

(a) fermenting unicellular host organisms transformed with an expression plasmid capable of expressing the said immunotoxin hybrid protein; and

(b) purifying the said immunotoxin hybrid protein by procedures known in the art.

3. A process for the preparation of a unicellular host organism capable of expressing an immunotoxin hybrid protein as defined in claim 1 or 2 which process comprises transforming a unicellular host organism in a manner known per se with a recombinant vector containing a DNA coding for the said immunotoxin hybrid protein.

4. A process for the preparation of a pharmaceutical composition containing an immunotoxin hybrid protein as defined in claim 1 or 2, which process comprises mixing the said immunotoxin hybrid protein with a compatible pharmaceutically acceptable carrier material.

5. A pharmaceutical composition comprising an immunotoxin hybrid protein as defined in claim 1 or 2 and a pharmaceutically acceptable carrier.

6. Use of an immunotoxin hybrid protein as defined in claim 1 or 2 for the preparation of a pharmaceutical composition.

7. An immunotoxin hybrid protein prepared by a process according to claim 1 or 2.

8. A unicellular host organism capable of expressing an immunotoxin hybrid protein as defined in claim 1 or 2 prepared by a process according to claim 3.

9. The invention as made and hereinbefore described.

10. A DNA coding for an immunotoxin hybrid protein as defined in claim 1 or 2.

11. A recombinant vector containing a DNA coding for an immunotoxin hybrid protein as defined in claim 1 or 2, which recombinant vector is capable of directing the expression of the DNA in a compatible unicellular host organism.

12. A unicellular host organism transformed with a recombinant vector comprising a DNA coding for an immunotoxin hybrid protein as defined in claim 1 or 2, which unicellular host organism is capable of expressing an immunotoxin hybrid protein as defined in claim 1 or 2.

# FIG. 1

Fig. 2

```
 1                                    10                                   20
H2N-Ala-Asp-Pro-Thr-Ser-Ser-Ser-Thr-Lys-Lys-Thr-Gln-Leu-Gln-Leu-Glu-His-Leu-Leu-Leu-
                                     30                                   40
     Asp-Leu-Gln-Met-Ile-Leu-Asn-Gly-Ile-Asn-Asn-Tyr-Lys-Asn-Pro-Lys-Leu-Thr-Arg-Met
                                     50                                   60
     Leu-Thr-Phe-Lys-Phe-Tyr-Met-Pro-Lys-Lys-Ala-Thr-Glu-Leu-Lys-His-Leu-Gln-Cys-Leu
                                     70                                   80
     Glu-Glu-Glu-Leu-Lys-Pro-Leu-Glu-Glu-Val-Leu-Asn-Leu-Ala-Gln-Ser-Lys-Asn-Phe-His
                                     90                                   100
     Leu-Arg-Pro-Arg-Asp-Leu-Ile-Ser-Asn-Ile-Asn-Val-Ile-Val-Leu-Glu-Leu-Lys-Gly-Ser
                                     110                                  120
     Glu-Thr-Thr-Phe-Met-Cys-Glu-Tyr-Ala-Asp-Glu-Thr-Ala-Thr-Ile-Val-Glu-Phe-Leu-Asn
                                     130                                  140
     Arg-Trp-Ile-Thr-Phe-Cys-Gln-Ser-Ile-Ile-Ser-Thr-Ile-Pro-Glu-Glu-Gly-Ser-Leu-Ala
                                     150                                  160
     Ala-Leu-Thr-Ala-His-Gln-Ala-Cys-His-Leu-Pro-Leu-Glu-Thr-Phe-Thr-Arg-His-Arg-Gln
                                     170                                  180
     Pro-Arg-Gly-Trp-Glu-Gln-Leu-Glu-Gln-Cys-Gly-Tyr-Pro-Val-Gln-Arg-Leu-Val-Ala-Leu
                                     190                                  200
     Tyr-Leu-Ala-Ala-Arg-Leu-Ser-Trp-Asn-Gln-Val-Asp-Gln-Val-Ile-Arg-Asn-Ala-Leu-Ala
                                     210                                  220
     Ser-Pro-Gly-Ser-Gly-Gly-Asp-Leu-Gly-Glu-Ala-Ile-Arg-Glu-Gln-Pro-Glu-Gln-Ala-Arg
                                     230                                  240
     Leu-Ala-Leu-Thr-Leu-Ala-Ala-Ala-Glu-Ser-Glu-Arg-Phe-Val-Arg-Gln-Gly-Thr-Gly-Asn
                                     250                                  260
     Asp-Glu-Ala-Gly-Ala-Ala-Asn-Ala-Asp-Val-Val-Ser-Leu-Thr-Cys-Pro-Val-Ala-Ala-Gly
                                     270                                  280
     Glu-Cys-Ala-Gly-Pro-Ala-Asp-Ser-Gly-Asp-Ala-Leu-Leu-Glu-Arg-Asn-Tyr-Pro-Thr-Gly
                                     290                                  300
     Ala-Glu-Phe-Leu-Gly-Asp-Gly-Gly-Asp-Val-Ser-Phe-Ser-Thr-Arg-Gly-Thr-Gln-Asn-Trp
                                     310                                  320
     Thr-Val-Glu-Arg-Leu-Leu-Gln-Ala-His-Arg-Gln-Leu-Glu-Glu-Arg-Gly-Tyr-Val-Phe-Val
                                     330                                  340
     Gly-Tyr-His-Gly-Thr-Phe-Leu-Glu-Ala-Ala-Gln-Ser-Ile-Val-Phe-Gly-Gly-Val-Arg-Ala
                                     350                                  360
     Arg-Ser-Gln-Asp-Leu-Asp-Ala-Ile-Trp-Arg-Gly-Phe-Tyr-Ile-Ala-Gly-Asp-Pro-Ala-Leu
                                     370                                  380
     Ala-Tyr-Gly-Tyr-Ala-Gln-Asp-Gln-Glu-Pro-Asp-Ala-Arg-Gly-Arg-Ile-Arg-Asn-Gly-Ala
                                     390                                  400
     Leu-Leu-Arg-Val-Tyr-Val-Pro-Arg-Ser-Ser-Leu-Pro-Gly-Phe-Tyr-Arg-Thr-Ser-Leu-Thr
                                     410                                  420
     Leu-Ala-Ala-Pro-Glu-Ala-Ala-Gly-Glu-Val-Glu-Arg-Leu-Ile-Gly-His-Pro-Leu-Pro-Leu
                                     430                                  440
     Arg-Leu-Asp-Ala-Ile-Thr-Gly-Pro-Glu-Glu-Glu-Gly-Gly-Arg-Leu-Glu-Thr-Ile-Leu-Gly
                                     450                                  460
     Trp-Pro-Leu-Ala-Glu-Arg-Thr-Val-Val-Ile-Pro-Ser-Ala-Ile-Pro-Thr-Asp-Pro-Arg-Asn
                                     470                                  480
     Val-Gly-Gly-Asp-Leu-Asp-Pro-Ser-Ser-Ile-Pro-Asp-Lys-Glu-Gln-Ala-Ile-Ser-Ala-Leu
                                     490                         496
     Pro-Asp-Tyr-Ala-Ser-Gln-Pro-Gly-Lys-Pro-Pro-Arg-Glu-Asp-Leu-Lys-COOH.
```

# Fig. 3

(75%)
Ala-Pro-Thr-Ser-Ser-Ser-Thr-Lys-Lys-Thr-Gln-Leu-Gln-Leu-Glu

(25%)
Met-Ala-Pro-Thr-Ser-Ser-Ser-Thr-Lys-Lys-Thr-Gln-Leu-Gln-Leu-Glu

# Fig. 4

| | |
|---|---|
| *E. coli* Cell Paste<br>Sonification | 4xvol 50mM Tris-HCl,<br>1mM EDTA, pH 8.0 |
| Pellet<br>Extraction | 5xvol 7M GuHCl, 0.1M Tris-HCl,<br>pH 8.0, 1mM ea.EDTA and DTT |
| Supernatant<br>Supernatant | 1-80 Dilution with PBS<br>Stir Overnight, Clarify |
| Receptor-Affinity<br>Chromatography | Wash Buffer. PBS Elution Buffer 3M KSCN<br>in 50mM Potassium Phosphate, pH 6.0 |
| Cation Exchange<br>CM-Fast Flow | 50mM Acetate, pH 5.0, Elution with 0.5M<br>NaCl in 50mM Potassium Phosphate, pH 6.0 |
| Sizing Column<br>Sepharose CL-6B | Mobile Phase: 50mM Potassium<br>Phosphate, pH 6.0, 0.15M NaCl |
| Concentration/<br>Diafiltration | Stored Frozen at -20°C |